(19) **Europäisches Patentamt / European Patent Office / Office européen des brevets**

(11) **EP 4 292 634 B1**

(12) **EUROPEAN PATENT SPECIFICATION**

(45) Date of publication and mention
of the grant of the patent:
**18.06.2025 Bulletin 2025/25**

(51) International Patent Classification (IPC):
***A61M 16/12*** (2006.01)   ***A61M 16/00*** (2006.01)
***A61M 16/16*** (2006.01)

(21) Application number: **23764219.4**

(22) Date of filing: **10.03.2023**

(52) Cooperative Patent Classification (CPC):
(C-Sets available)
**A61M 16/12; A61M 16/0051; A61M 16/026;**
**G16H 20/13; G16H 20/40; G16H 40/63;**
**G16H 50/70;** A61M 11/00; A61M 16/14;
A61M 2016/0027; A61M 2016/003;
A61M 2202/0208; A61M 2202/0225;
A61M 2202/0484; A61M 2205/18;   (Cont.)

(86) International application number:
**PCT/CN2023/080732**

(87) International publication number:
**WO 2023/185415 (05.10.2023 Gazette 2023/40)**

(54) **VENTILATOR FOR TREATING ALCOHOL INTOXICATION BASED ON GAS MIXING RATIO OF OXYGEN AND CARBON DIOXIDE**

BEATMUNGSGERÄT ZUR BEHANDLUNG VON ALKOHOLVERGIFTUNGEN BASIEREND AUF DEM GASMISCHUNGSVERHÄLTNIS VON SAUERSTOFF UND KOHLENDIOXID

VENTILATEUR POUR LE TRAITEMENT D'INTOXICATION ALCOOLIQUE SUR LA BASE DU RAPPORT DE MÉLANGE DE GAZ D'OXYGÈNE ET DE DIOXYDE DE CARBONE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **01.04.2022 CN 202210348423**

(43) Date of publication of application:
**20.12.2023 Bulletin 2023/51**

(73) Proprietor: **Guangzhou Landswick Medical Technologies Ltd.**
**Guangzhou, Guangdong 510000 (CN)**

(72) Inventors:
• **DONG, Hui**
**Guangzhou, Guangdong 510000 (CN)**
• **LIN, Xiaomei**
**Guangzhou, Guangdong 510000 (CN)**
• **ZHAO, Longchao**
**Guangzhou, Guangdong 510000 (CN)**
• **SUN, Caixin**
**Guangzhou, Guangdong 510000 (CN)**

(74) Representative: **Ipside**
**7-9 Allée Haussmann**
**33300 Bordeaux Cedex (FR)**

(56) References cited:
EP-A1- 3 666 279      CN-A- 105 748 069
CN-A- 106 139 343    CN-A- 110 269 988
CN-A- 111 658 932    CN-A- 113 289 182
CN-A- 113 663 198    CN-A- 114 588 467
CN-U- 208 710 724    KR-A- 980 008 903
SU-A1- 1 296 157     US-A- 3 974 830
US-A1- 2013 118 484  US-A1- 2020 405 823

(52) Cooperative Patent Classification (CPC): (Cont.)
A61M 2205/3331; A61M 2205/3334;
A61M 2205/6009; A61M 2230/06; A61M 2230/20;
A61M 2230/202; A61M 2230/205; A61M 2230/30;
A61M 2230/42

C-Sets
A61M 2202/0208, A61M 2202/0007;
A61M 2202/0225, A61M 2202/0007;

A61M 2202/0484, A61M 2202/0085;
A61M 2230/06, A61M 2230/005;
A61M 2230/202, A61M 2230/005;
A61M 2230/205, A61M 2230/005;
A61M 2230/30, A61M 2230/005;
A61M 2230/42, A61M 2230/005

## Description

## Technical Field

The present disclosure relates to the technical field of treating alcohol intoxication with a ventilator, and more particular to a method for treating alcohol intoxication by supplying gases based on a gas mixing ratio and a ventilator therefor.

## Background of the Disclosure

Alcohol intoxication is commonly known as: alcohol poisoning, and drunkenness is mild alcohol intoxication. Alcohol intoxication refers to the physical effects and negative behavior of a person as a result of recent consumption of alcohol. Symptoms of alcohol intoxication at low doses include mild sedative effects and poor coordination. Higher doses may cause slurred speech, difficulty walking, and vomiting. Excessive alcohol may cause breathing difficulties, coma, and even death. Some possible complications include seizures, aspiration pneumonia, physical trauma (including suicide), and hypoglycemia. Alcohol is broken down in the human body at a rate of approximately 3.3 mmol/L (15mg/dL) per hour.

Patent document CN106139343A discloses a gassing first-aid apparatus in the medical instruments field. The apparatus includes an air admission unit, gas mixing units, gas feed unit, and control unit. Gas passes through the air admission unit, then the gas mixing units and the gas feed unit in sequence, with the control unit connected to both. The control unit monitors the gas composition in real-time and adjusts the gas mixing units and gas feed unit as needed. This apparatus addresses the limitations of existing gassing first-aid equipment by providing adaptability to different respiratory rates and the supply of oxygen and carbon dioxide gas mixtures.

Patent document CN111658932A discloses provides a respirator for spontaneous respiration stimulation tests, utilizing a gas mixing controller to mix high-pressure carbon dioxide and oxygen in precise proportions for continuous mechanical ventilation. This method eliminates the need for multiple blood draws, increases test success rates, and improves patient tolerance.

Patent document US3974830A discloses a therapeutic technique known as Carbon Dioxide Therapy (CDT), which is utilized in the treatment of various addictions. The patent outlines a specific methodology for administering controlled doses of carbon dioxide to a patient, along with the necessary equipment designed to facilitate this process. The apparatus includes mechanisms for the safe delivery and monitoring of CO2 concentrations to ensure the patient's well-being during the therapy sessions. This innovative approach to addiction treatment focuses on altering the biochemical processes associated with addiction and aims to reduce the patient's dependency on addictive substances.

Patent document CN208710724U describes a breathing equipment comprising a mask with gas concentration level monitor and controller, and an inlet duct with input pipe, mixing tube, oxygen conveying pipe, and carbon dioxide delivery pipe. The controller adjusts the carbon dioxide content in the gas by regulating the valve, providing suitable carbon dioxide levels for the patient to balance blood pH and reduce symptoms. An atomizer adds moisture to the gas, improving patient comfort.

Patent document CN105748069A discloses a carbon dioxide inhalation therapy device for central sleep apnea. It includes an air blower, a gas bottle with carbon dioxide, an air bag, a mask, and a detection mechanism using electrical signals to detect central sleep apnea. The mask has through holes for airflow, connected to the air bag and air blower via pipelines, with the gas bottle connected through a flowmeter. This device provides stable and moderate carbon dioxide to correct central sleep apnea without increasing arousal frequency.

Patent document SU1296157 A1 describes a method for treating acute alcohol intoxication using hyperbaric oxygen therapy to reduce treatment time without adverse effects. This method aims to significantly shorten the time required for treatment compared to traditional methods, which typically take 2.5 to 3 hours to achieve sobriety. The process is reported to normalize physiological indicators such as skin coloration, respiratory rate, blood pressure, and pulse, as well as improve coordination and cognitive function without causing any side effects or complications.

The approach to alcohol intoxication is usually supportive. Close monitoring; prevention of breathing or choking; oxygen therapy; fluids given through an IV (intravenous); to prevent dehydration; vitamins and glucose to help prevent serious complications of alcohol intoxication. Some methods, such as gastric lavage or activated charcoal, do not have any evidence of benefit. Repeated judgment may be required during the recovery process to avoid any cause of other symptoms. Also, adults and children who drink methanol or isopropanol may need hemodialysis (a mechanical method of filtering waste and toxins from the patient's body to speed up the removal of alcohol from the blood)

However, the treatment of alcohol intoxication in the prior art is not standardized, and there is currently no special equipment for the treatment of alcohol intoxication.

Therefore, the present disclosure provides a method and ventilator for solving alcohol poisoning based on the mixing ratio of the mixed gas. By using different ratios of carbon dioxide $CO_2$ and oxygen $O_2$ to supply gas, the metabolism of alcohol can be accelerated, the alcohol in the body can be quickly removed, and excessive alcohol can be avoided at the same time. Respiratory alkalosis caused by ventilation improves resolution efficiency and safety in alcohol intoxication.

## Summary of the Invention

The present invention provides a ventilator for treating alcohol intoxication based on gas mixing ratio as defined in claim 1.

## Summary of the Disclosure

Reference will now be made to various exemplary embodiments or aspects of the present disclosure. Embodiments or aspects disclosed herein which do not fall under the scope of the appended claim 1 do not form part of the present invention, but are useful for understanding the principles of the invention.

The scope of the present invention is defined by the appended claim 1.

The present disclosure provides a method (not claimed) and ventilator for solving alcohol poisoning based on gas mixing ratio gas supply, which is used to use different ratios of carbon dioxide $CO_2$ and oxygen $O_2$ to supply gas, which can quickly remove alcohol in the body, and at the same time avoid breathing caused by hyperventilation Alkalosis improves the efficiency and safety of solving alcohol intoxication.

The present disclosure provides a method for solving alcohol poisoning by supplying gas based on the gas mixing ratio, comprising:

Step (1): mixing oxygen and carbon dioxide gas to generate a target gas mixture, and transmitting the target gas mixture to the subject based on a preset breathing device;
Step (2): monitoring the alcohol concentration in the subject in real time, and at the same time, monitor monitoring the partial pressure of carbon dioxide and the respiratory rate in the subject;
Step (3): on the basis of the stable partial pressure of carbon dioxide and respiratory rate of the subject, when the alcohol concentration in the subject meets the safety range, controlling the preset breathing device to stop working.

Preferably, a method for solving alcohol poisoning by supplying gas based on a gas mixing ratio, in step (3), mixing oxygen and carbon dioxide gas to generate a target mixed gas, comprising:

Collecting the current alcohol concentration of the subject, and at the same time, determining the current body characteristic index of the subject;
Inputting the current alcohol content concentration of the subject and the current body characteristic index of the subject into the alcohol analysis model for analysis;
Based on the analysis results in the alcohol analysis model, determine the oxygen concentration and carbon dioxide concentration required by the subject respectively;
Based on the oxygen concentration and the carbon dioxide concentration, determine the mixing ratio of the oxygen and carbon dioxide;
Based on the mixing ratio, mix the oxygen and the carbon dioxide to generate the target mixed composition.

Preferably, a method for solving alcohol poisoning by supplying air based on the gas mixing ratio, in step (1), transmitting the target mixed gas to the subject based on a preset breathing device, further comprising:

reading the identity information of the subject, and determine the test number and name of the subject;
matching the test number of the subject and the name of the subject with the registration information, and judging whether the target gas mixture can be transmitted to the subject based on the preset breathing device;
when the test number of the subject and the name of the subject do not match the registration information, the target gas mixture cannot be transmitted to the subject based on the preset breathing device; and
when the test number of the subject and the name of the subject match the registration information, a gas transmission instruction is generated, and based on the gas transmission instruction, the preset breathing device is controlled to transmit the target gas mixture to the subject.

Preferably, a method for solving alcohol poisoning based on gas mixing ratio gas supply, in step (2), real-time monitroring of the alcohol concentration in the body of the subject, and at the same time, monitoring the partial pressure of carbon dioxide and respiratory rate of the subject, comprising:

when the target gas mixture is transmitted to the subject based on the preset breathing device, starting a first monitoring instruction and a second monitoring instruction;

monitoring the dynamic alcohol concentration in the subject in real time based on the first monitoring instruction;

monitoring the partial pressure of carbon dioxide in the subject and the respiratory rate of the subject in real time based on the second monitoring instruction;

according to the preset time point, recording the dynamic alcohol change concentration in one-to-one correspondence with the partial pressure of carbon dioxide and the respiratory rate in the subject; and

based on the recorded results, a monitoring dynamic table is generated and displayed in real time.

Preferably, a method for solving alcohol poisoning by supplying gas based on the gas mixing ratio, based on the recorded results.

After the monitoring dynamic table is generated, it also comprises:

reading the monitoring dynamic table to determine the numerical mapping relationship between the dynamic concentration of alcohol in the subject and the preset time point under the condition that the mixing ratio of the carbon dioxide and the oxygen concentration remains unchanged ;establishing a target Cartesian coordinate system based on the numerical mapping relationship, and determine the numerical mapping curve;

reading the numerical mapping curve, determine the change trend of the dynamic alcohol concentration in the subject, and at the same time, according to the change trend of the dynamic alcohol concentration in the subject, determine the change in the numerical value target inflection points in the map graph;

determining the target alcohol concentration in the subject corresponding to the target inflection point, and comparing the target alcohol concentration with the alcohol concentration safety range;

when the target alcohol concentration is within the safety range of the alcohol concentration, completing the decomposition of the alcohol concentration in the subject;

when the target alcohol concentration is not within the safe range of alcohol concentration, adjusting the mixing ratio of the mixed gas of oxygen and carbon dioxide based on the target alcohol concentration in the subject until the target alcohol concentration in the subject until the alcohol concentration is within the safe range of alcohol concentration.

Preferably, a method for solving alcohol poisoning by supplying gas based on the gas mixing ratio, the process of adjusting the mixing ratio of the mixed gas of oxygen and carbon dioxide comprises:

gradually changing concentration of the carbon dioxide according to the preset concentration range, and mixing the carbon dioxide and oxygen according to the gradient change, and the adjustment of the mixing ratio of the mixed gas of oxygen and carbon dioxide is completed; wherein the gradient change in the preset concentration change range is: the concentration of carbon dioxide changing in gradient from 1% to 6%.

Preferably, a method for solving alcohol poisoning by supplying air based on the gas mixing ratio, monitoring the partial pressure of carbon dioxide and the breathing rate in the subject in real time based on the second monitoring instruction, further comprising:

recording the partial pressure of carbon dioxide in the subject and the breathing rate of the subject in real time;

respectively comparing the partial pressure of carbon dioxide in the subject with the reference partial pressure of carbon dioxide range, and comparing the respiratory rate of the subject with the reference respiratory rate range, and obtaining comparison results;

judging whether to generate an alarm instruction based on the comparison result;

wherein in the comparison result, when the partial pressure of carbon dioxide in the subject is within the range of the reference partial pressure of carbon dioxide, and the respiratory rate of the subject is within the range of the reference respiratory rate, then determining not to generate an alarm command;

when the partial pressure of carbon dioxide in the subject is not within the range of the reference partial pressure of carbon dioxide, and the respiratory rate of the subject is within the range of the reference respiratory rate, a first alarm instruction is generated, and based on the first alarm command is used to perform the first alarm operation;

when the partial pressure of carbon dioxide in the subject is within the range of the reference partial pressure of carbon dioxide, but the respiratory rate of the subject is not in the range of the reference respiratory rate, a second alarm instruction is generated, and based on the second alarm command is used to perform the second alarm operation;

otherwise, generate a first alarm instruction and a second alarm instruction at the same time, and perform a third alarm operation based on the first alarm instruction and the second alarm instruction.

Preferably, a method for solving alcohol poisoning by supplying air based on the gas mixing ratio, in step (3), when the alcohol concentration in the subject meets a safe range, controlling the preset breathing device to stop working comprises:

Extracting the alcohol concentration threshold in the safe range, and using the alcohol concentration threshold as a reference signal to generate a control instruction;

The alcohol concentration in the subject is monitored in real time, and when the alcohol concentration in the subject is equal to the alcohol concentration threshold, the control instruction is triggered to control the preset breathing device to stop working.

Preferably, a method for solving alcohol poisoning by supplying gas based on the gas mixing ratio, in step (2), thealcohol content concentration and the current body characteristic index of the subject are input into the alcohol analysis model for analysis, specifically comprising:

reading several mixed gas alcohol concentration elimination cases, and extracting case data, and one mixed gas alcohol concentration elimination case corresponds to a target subject;

inputting the case data into the preset neural network as sample data, and an iteration factor is determined according to the content of the sample data;

performing data iterative processing on the sample data based on the iteration factor, and determining data feature points of the sample data based on the iteration result of the preset neural network; wherein the data feature points comprise: the identity information of several target subjects, the body characteristic index of the target subject and the mixing ratio of the mixed gas inhaled by the target subject;

classifying the sample data based on the data feature points, and at the same time, determining a first category vector, a second category vector, and a third category vector according to the classification result;

respectively determining a first association relationship between the first category vector and the second category vector, a second association relationship between the first category vector and the third category vector, and the third association of vectors between the second category vector and the third category vector;

constructing the alcohol analysis model based on the first correlation, the second correlation and the third correlation; extracting the identity information of the subject and the body condition index of the subject, and inputting the identity information of the subject and the body condition index of the subject into the alcohol analysis model;

based on the alcohol analysis model, determining a first weight value corresponding to the subject's identity information and a second weight value corresponding to the subject's physical condition; and in the alcohol analysis model, based on the first weight value and the second weight value, outputting the mixing ratio of the mixed gas required by the subject.

Preferably, a ventilator for solving alcohol poisoning based on gas mixing ratio, comprising:

The gas transmission module is used to mix oxygen and carbon dioxide gas to generate a target mixed gas, and transmitting the target gas mixture to the subject based on the preset breathingdevice;

The monitoring module is used to monitor the alcohol concentration in the subject in real time, and at the same time, monitor the partial pressure of carbon dioxide and the respiratory rate in the subject;

The control module is used to control the preset breathing device to stop working when the alcohol concentration in the subject meets a safe range on the basis of the subject's partial pressure of carbon dioxide and respiratory rate being stable.

Other features and advantages of the present disclosure will be set forth in the following description, and partly become apparent from the description, or can be understood by implementing the present disclosure. The objectives and other advantages of the disclosure may be realized and attained by the structure particularly pointed out in the written description and claims hereof as well as the appended drawings.

The technical solutions of the present disclosure will be described in further detail below with reference to the accompanying drawings and embodiments.

## Brief description of the Drawings

The accompanying drawings are used to provide a further understanding of the present disclosure, and constitute a part of the description, and are used to explain the present disclosure together with the embodiments of the present disclosure, and do not constitute a limitation to the present disclosure. In the attached picture:

Fig. 1 is a flow chart of a method for solving alcohol poisoning by supplying gas based on the gas mixing ratio in an embodiment of the present disclosure;

Fig. 2 is a schematic diagram of a method for solving alcohol poisoning by supplying gas based on the gas mixing ratio in an embodiment of the present disclosure;

Fig. 3 is a structural diagram of solving alcohol mixed gas based on the gas mixing ratio in the embodiment of the present disclosure;

Fig. 4 is a structural diagram of the ventilator system device in the embodiment of the present disclosure;

Fig. 5 is a histogram of the impact of acute severe alcohol intoxication on heart rate in an embodiment of the present disclosure;

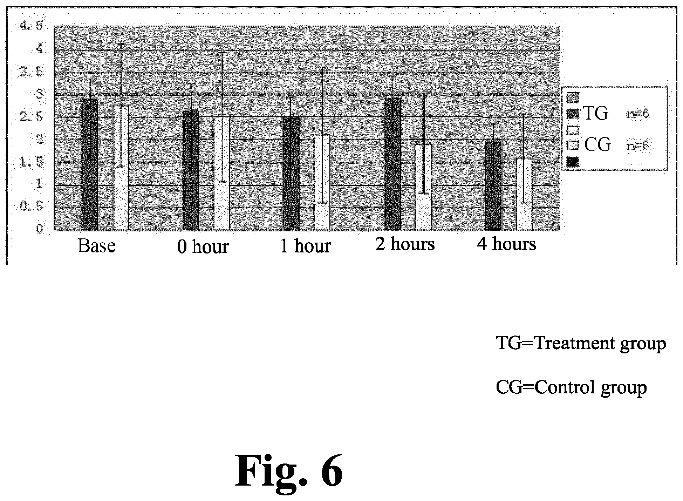

Fig. 6 is a histogram of the impact of acute severe alcohol intoxication on CI in an embodiment of the present disclosure;

Fig. 7 is a histogram of the impact of acute severe alcohol intoxication in an embodiment of the present disclosure The histogram of the influence of alcohol intoxication on SVI;

Fig. 8 is the histogram of the influence of acute severe alcohol intoxication on MAP in the embodiment of the present disclosure;

Fig. 9 is the histogram of the influence of PCO2 in the embodiment of the present disclosure;

Fig. 10 is a histogram of the effect on awake time in an embodiment of the present disclosure.

## Detailed Description of Embodiments

The following embodiments 3 and 5-10 are not according to the invention and are present for illustration purposes only. The preferred embodiments of the present invention will be described below in conjunction with the accompanying drawings. It should be understood that the preferred embodiments described here are only used to illustrate and explain the present invention, and are not intended to limit the present invention.

### Embodiment 1

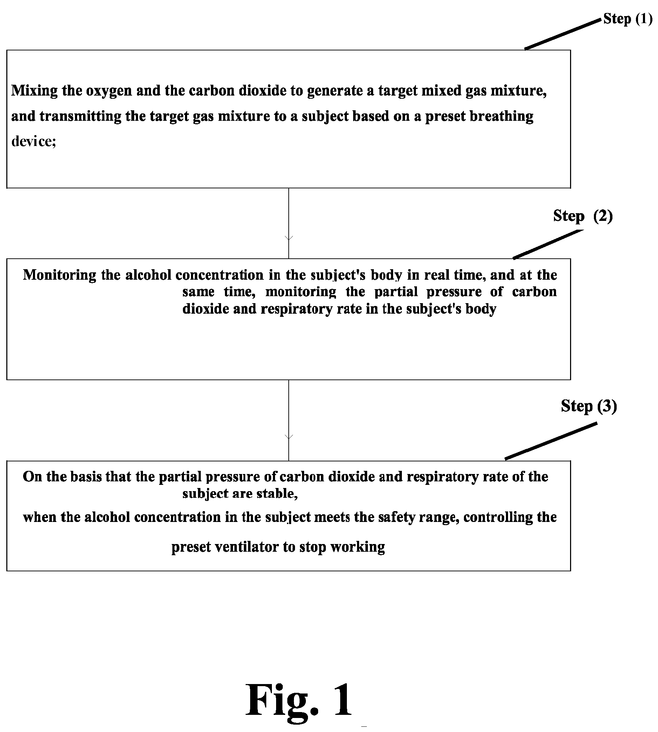

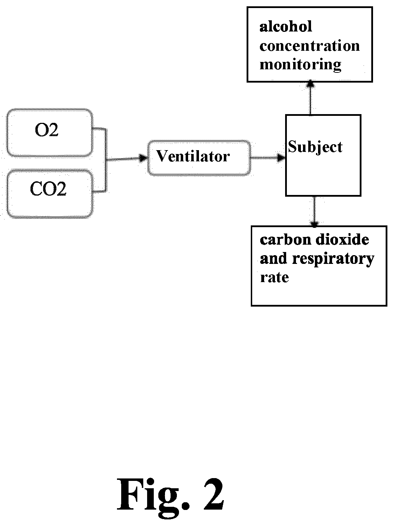

This embodiment provides a method for solving alcohol poisoning by supplying gas based on the gas mixing ratio, as shown in Figs. 1-2, comprising:

Step (1): mixing oxygen and carbon dioxide gas to generate a target gas mixture, and transmitting the target gas mixture to the subject based on a preset breathing device;
Step (2): monitoring the alcohol concentration in the subject in real time, and at the same time, monitor the partial pressure of carbon dioxide and the respiratory rate in the subject;
Step (3): on the basis of the stable partial pressure of carbon dioxide and respiratory rate of the subject, when the alcohol concentration in the subject meets the safety range, controlling the preset breathing device to stop working.

In this embodiment, the target mixed gas is generated based on a certain mixing ratio of oxygen and carbon dioxide, and is used to eliminate the alcohol concentration in the subject.

In this embodiment, 1) a certain proportion of $CO_2$ gas is injected into pure oxygen to treat alcohol intoxication; 2) the treatment process is simplified to improve patient comfort; 3) the ventilator with this function is used to reduce the cost of equipment.

In this embodiment, the data of animal clinical experiments show that when the volume ratio of oxygen and $CO_2$ is 94%: 6%, it can inhibit the reduction of alcohol poisoning on cardiac output, thereby accelerating the metabolism of alcohol; the pressure is maintained at a normal level without increasing carbon dioxide retention; the carbon dioxide gas mixed ventilator has a wake-up effect on the disturbance of consciousness caused by acute severe alcohol intoxication.

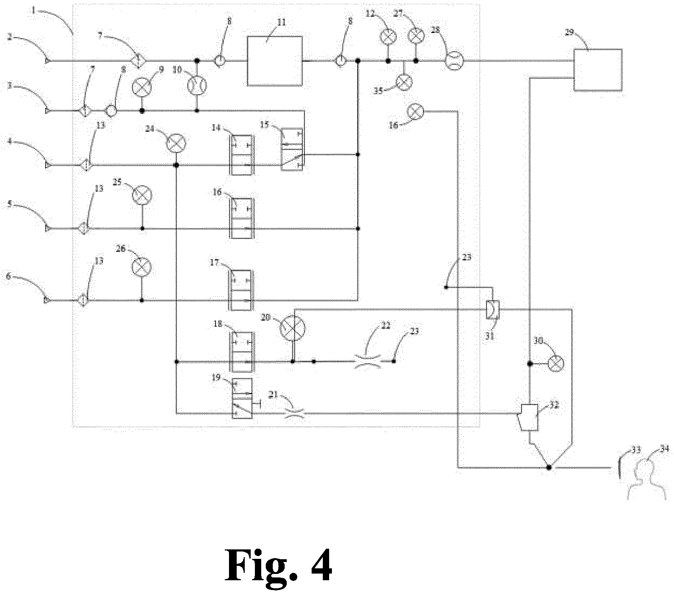

In this embodiment, the ventilator system device is shown in Fig. 4:
Comprising: ventilator system device body 1, air source inlet 2, low-pressure oxygen source 3, high-pressure oxygen source 4, first treatment gas port 5, second treatment gas port 6, first filter 7, check valve 8 , first pressure sensor 9 first flow sensor 10, blower 11, second pressure sensor 12, second filter 13, first flow control valve 14, directional control valve 15, second flow control valve 16, third flow control valve 17 , the fourth flow control valve 18, the on-off valve 19, the third pressure sensor 20, the first throttle valve 21, the second throttle valve 22, the pipeline outlet 23, the second pressure sensor 24, the second pressure sensor 25, the second Two pressure sensors 26, oxygen sensor 27, the second flow sensor 28, heating humidifier 29, respiratory pressure sensor 30, exhaust valve 31, medicine nebulizer 32, breathing tube 33, suction pipeline 34, oxygen (or carbon dioxide ) concentration sensor 35.

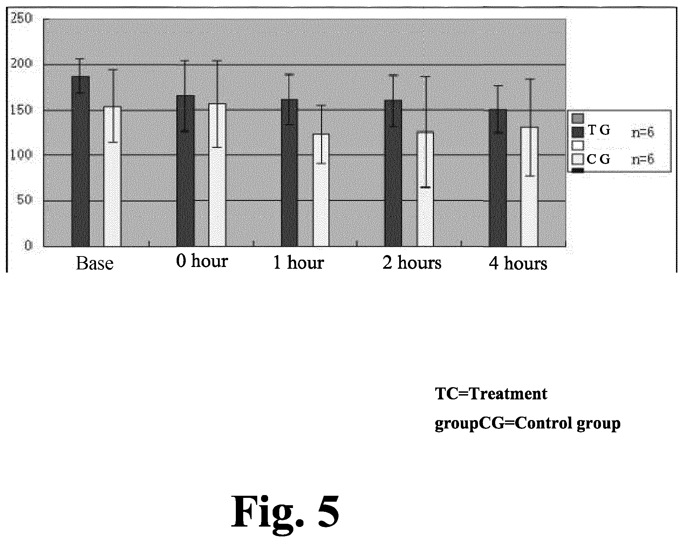

In this embodiment, as shown in Fig. 5, the impact of acute severe alcohol intoxication on heart rate (compared with the 4-hour group in the control group, P value>0.05, no statistical significance compared with the 4-hour group in the experimental group, P Value <0.05, statistically significant △P between groups > 0.05, no statistical significance. It shows that carbon dioxide mixed gas can increase the heart rate compared with the control group in the treatment of acute severe alcohol intoxication.)

In this embodiment, as shown in 6, the impact of acute severe alcohol intoxication on CI (compared with the 4-hour group in the experimental group, P value>0.05, no statistical significance compared with the 4-hour group in the control group, P value <0.05, which is statistically significant. Compared between groups, △P>0.05, not statistically significant. It shows that the carbon dioxide mixed gas ventilator can reduce the decline of cardiac index compared with the control group in acute severe alcohol intoxication, and then promote the ethanol in the metabolic rate in the blood.)

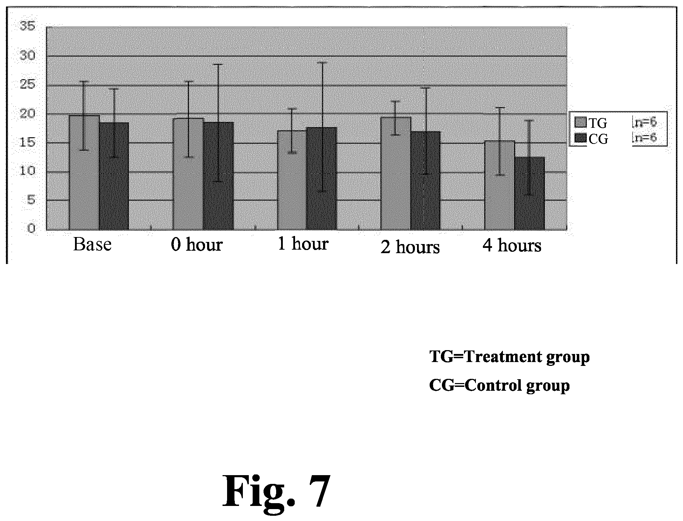

In this embodiment, as shown in Fig. 7, the impact of acute severe alcohol intoxication on SVI (compared with the 4-hour group in the control group, P value>0.05, no statistical significance compared with the 4-hour group in the experimental group, P Value>0.05, no statistical significance. $\triangle P>0.05$ between groups, no statistical significance. But the SVI of the control group decreased significantly, indicating that the use of carbon dioxide gas mixture does not reduce the stroke volume of the heart. )

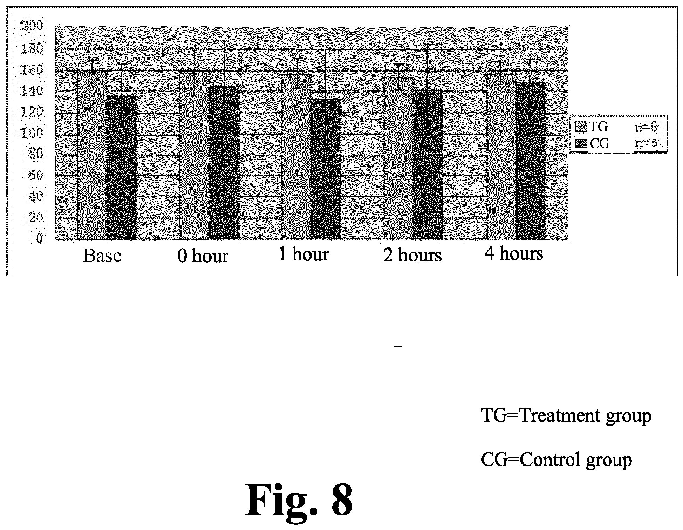

In this embodiment, as shown in Fig. 8, the impact of acute severe alcohol intoxication on MAP (compared with the 4-hour group in the control group, P value>0.05, no statistical significance compared with the 4-hour group in the experimental group, P Value>0.05, no statistical significance $\triangle P$ between groups>0.05, no statistical significance. It shows that the use of carbon dioxide mixed gas has no effect on mean arterial pressure in acute severe alcohol intoxication.)

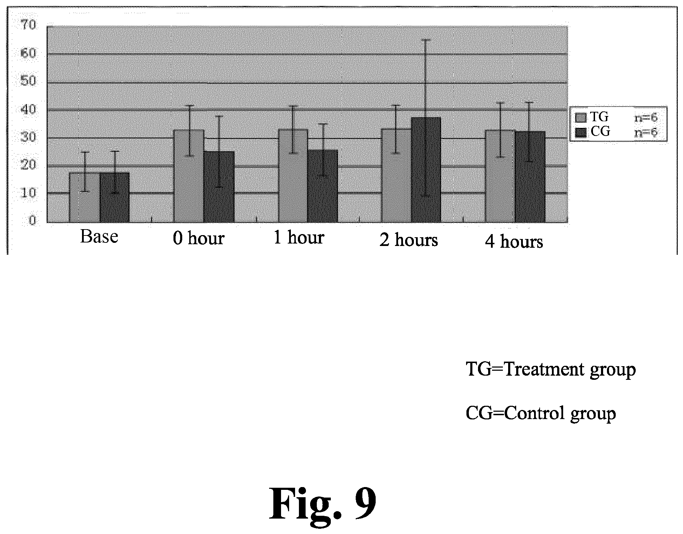

In this embodiment, as shown in Fig. 9, the effect on PCO2 (in the control group compared with the basal and 4-hour group Comparison, P value <0.05, statistically significant Comparing the base in the experimental group with the 4-hour group, P value <0.05, statistically significant $\triangle P>0.05$ between groups, no statistical significance. Explain that the carbon dioxide mixed gas will not cause carbon dioxide retention during use. )

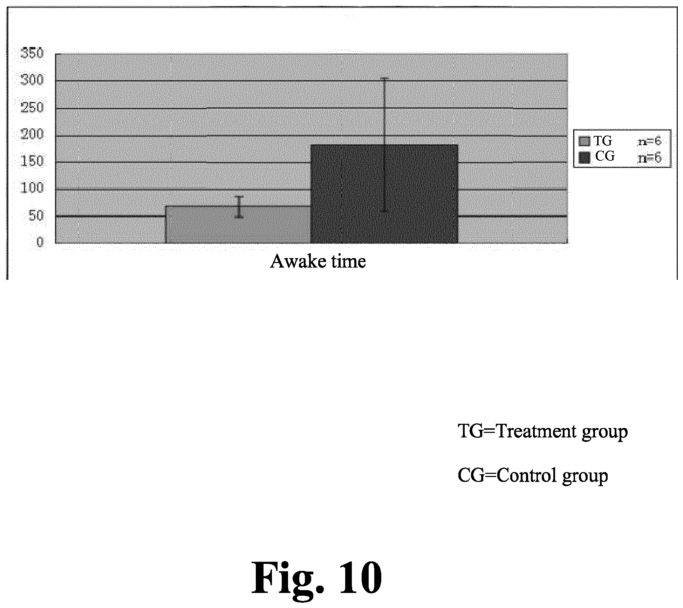

In this embodiment, as shown in Fig. 10, the impact on the waking time (compared with the control group, P<0.05, has statistical significance. It shows that the carbon dioxide mixed gas ventilator has a positive effect on the disturbance of consciousness caused by acute severe alcohol intoxication. Wake up.)

The beneficial effect of the above-mentioned technical solution is: the mixed gas supply of carbon dioxide $CO_2$ and oxygen $O_2$ in different proportions can accelerate the metabolism of alcohol, quickly remove the alcohol in the body, and at the same time avoid respiratory alkalosis caused by hyperventilation, and improve the solution to alcohol intoxication. efficiency and safety.

## Embodiment 2

On the basis of Embodiment 1, this embodiment provides a method for solving alcohol poisoning by supplying gas based on the gas mixing ratio. In step (1), oxygen and carbon dioxide are mixed to generate a target mixed gas, comprising:

Collecting the current alcohol concentration of the subject, and at the same time, determining the current body characteristic index of the subject;

Inputting the current alcohol content concentration of the subject and the current body characteristic index of the subject into the alcohol analysis model for analysis;

Based on the analysis results in the alcohol analysis model, determine the oxygen concentration and carbon dioxide concentration required by the subject respectively;

Based on the oxygen concentration and the carbon dioxide concentration, determine the mixing ratio of the oxygen and carbon dioxide;

Based on the mixing ratio, the oxygen and the carbon dioxide are mixed to generate the target mixed gas.

In this embodiment, the current body characteristic index comprises: the subject's blood pressure, heart rate, etc., which are used to characterize the subject's physical condition. The larger the index, the better the subject's physical condition.

In this embodiment, the alcohol analysis model can be used to determine the optimal oxygen and carbon dioxide concentrations according to the subject's current alcohol concentration and the subject's current body characteristic index.

The beneficial effect of the above technical solution is: by inputting the subject's current alcohol concentration and the subject's current physical characteristic index into the alcohol analysis model for analysis, it is beneficial to accurately determine the optimal oxygen and carbon dioxide for the subject The mixed concentration of the mixed gas improves the safety and accuracy of the subject's alcohol concentration elimination.

## Embodiment 3

On the basis of Embodiment 1, this embodiment provides a method for solving alcohol poisoning based on gas mixing ratio gas supply. In step (1), the target gas mixture is transmitting to the subject based on the preset breathing device, which also comprises:

Read the identity information of the subject, and determine the test number and name of the subject;

matching the test number of the subject and the name of the subject with the registration information, and judging whether the target gas mixture can be transmitted to the subject based on the preset breathing device;

When the test number of the subject and the name of the subject do not match the registration information, the target gas mixture cannot be transmitted to the subject based on the preset breathing device;

When the test number of the subject and the name of the subject match the registration information, a gas transmission instruction is generated, and based on the gas transmission instruction, the preset breathing device is controlled to transmit the target gas mixture to the subject.

In this embodiment, the subject's identity information comprises: subject's name, age, gender and other identity information, test number and other information, wherein the test number can be determined according to the ranking of the subjects.

In this embodiment, the registration information may be the registration of information that the subject needs to perform when eliminating the alcohol concentration.

In this embodiment, the purpose of matching the registration information with the subject's identity information is for the accuracy of the test and to avoid confusion in the test.

The beneficial effect of the above-mentioned technical solution is: verifying the identity information of the subject and the registration information of the subject helps to determine the accuracy of the test for the subject.

**Embodiment 4**

On the basis of Embodiment 1, this embodiment provides a method for solving alcohol poisoning based on the gas mixing ratio. In step (2), the alcohol concentration in the subject is monitored in real time, and at the same time, the subject's Partial pressure of carbon dioxide and respiratory rate, including:

When the target gas mixture is transmitted to the subject based on the preset breathing device, start a first monitoring instruction and a second monitoring instruction;

monitoring the dynamic alcohol concentration in the subject in real time based on the first monitoring instruction;

monitoring the partial pressure of carbon dioxide in the subject and the respiratory rate of the subject in real time based on the second monitoring instruction;

According to the preset time point, the dynamic alcohol change concentration is recorded in one-to-one correspondence with the partial pressure of carbon dioxide and the respiratory rate in the subject;

Based on the recorded results, a monitoring dynamic table is generated and displayed in real time.

In this embodiment, the first monitoring instruction may be used to monitor changes in alcohol concentration in the subject.

In this embodiment, the second monitoring instruction may be used to monitor the partial pressure of carbon dioxide in the subject's body and the breathing rate of the subject after the subject inhales the mixed gas.

The beneficial effect of the above-mentioned technical solution is: the first monitoring instruction helps to obtain the change of the alcohol content in the subject in real time, thereby helping to grasp the test data of the subject in real time and improving the accuracy of the test. The second monitoring instruction has It is beneficial to grasp the partial pressure of carbon dioxide and the respiratory rate of the subject in real time, so that the physical condition of the subject can be well grasped, and respiratory alkalosis caused by hyperventilation can be avoided.

**Embodiment 5**

On the basis of Embodiment 4, this embodiment provides a method for solving alcohol poisoning by supplying gas based on the gas mixing ratio. After the monitoring dynamic table is generated based on the recording results, it also comprises:

Reading the monitoring dynamic table to determine the numerical mapping relationship between the dynamic concentration of alcohol in the subject and the preset time point under the condition that the mixing ratio of the carbon dioxide and the oxygen concentration remains unchanged ;

And establish a target Cartesian coordinate system based on the numerical mapping relationship, and determine the numerical mapping curve;

Read the numerical mapping curve, determine the change trend of the dynamic alcohol concentration in the subject, and at the same time, according to the change trend of the dynamic alcohol concentration in the subject, determine the change in the numerical value target inflection points in the map graph;

Determine the target alcohol concentration in the subject corresponding to the target inflection point, and compare the target alcohol concentration with the alcohol concentration safety range;

When the target alcohol concentration is within the safety range of the alcohol concentration, the decomposition of the alcohol concentration in the subject is completed;

When the target alcohol concentration is not within the safe range of alcohol concentration, adjust the mixing ratio of the mixed gas of oxygen and carbon dioxide based on the target alcohol concentration in the subject until the target alcohol concentration in the subject The alcohol concentration is within the safe range of alcohol concentration.

In this embodiment, the numerical mapping relationship may be the change of the dynamic alcohol concentration as the preset time point changes.

In this embodiment, the preset time point is set in advance, for example, every five minutes is a time point, that is, 5 minutes, 10 minutes, etc. from the time when the subject inhales the mixed gas.

In this embodiment, the numerical mapping curve is determined based on the numerical mapping relationship, and the change status of the curve can be analyzed intuitively.

In this embodiment, the target inflection point may be a point used to represent a value change in the value mapping curve.

In this embodiment, the target alcohol concentration may be the alcohol concentration corresponding to the target inflection point.

In this embodiment, the safe range of alcohol concentration is set in advance.

The beneficial effect of the above-mentioned technical scheme is: by generating the dynamic alcohol concentration in the subject

Corresponding numerical mapping curves, and according to the numerical mapping curves, the inflection point of the alcohol concentration in the subject can be accurately judged, so as to facilitate the accurate judgment of the current alcohol concentration in the subject, and improve the efficiency of solving alcohol intoxication.

**Embodiment 6**

On the basis of Embodiment 5, this embodiment provides a method for solving alcohol poisoning by supplying gas based on the gas mixing ratio, the process of adjusting the mixing ratio of the mixed gas of oxygen and carbon dioxide, comprising:

Gradiently changing the concentration of the carbon dioxide according to the preset concentration range, and mixing the carbon dioxide and oxygen according to the gradient change, and completing the adjustment of the mixing ratio of the mixed gas of oxygen and carbon dioxide;

Wherein, the gradient change in the preset concentration change range is: the concentration of carbon dioxide is changed in gradient from 1% to 6%.

In this embodiment, the preset concentration range is set in advance.

The beneficial effect of the above technical solution is: by adjusting the concentration of carbon dioxide in a gradient according to the preset concentration range, it is convenient to quickly and effectively solve the alcohol in the subject's body, and the efficiency and safety of alcohol intoxication are improved.

**Embodiment 7**

On the basis of Embodiment 4, this embodiment provides a method for solving alcohol poisoning by supplying gas based on the gas mixing ratio, and monitoring the partial pressure of carbon dioxide and the respiratory rate in the subject in real time based on the second monitoring instruction, further comprising:

recording the partial pressure of carbon dioxide in the subject and the breathing rate of the subject in real time;

respectively comparing the partial pressure of carbon dioxide in the subject with the reference partial pressure of carbon dioxide range, and comparing the respiratory rate of the subject with the reference respiratory rate range, and obtaining comparison results;

judging whether to generate an alarm instruction based on the comparison result;

wherein, in the comparison result, when the partial pressure of carbon dioxide in the subject is within the range of the reference partial pressure of carbon dioxide, and the respiratory rate of the subject is within the range of the reference respiratory rate, then Determine not to generate an alarm command; when the partial pressure of carbon dioxide in the subject is not within the range of the reference partial pressure of carbon dioxide, and the respiratory rate of the subject is within the range of the reference respiratory rate, a first alarm instruction is generated, and based on the first alarm command is used to perform the first alarm operation;

when the partial pressure of carbon dioxide in the subject is within the range of the reference partial pressure of carbon dioxide, but the respiratory rate of the subject is not in the range of the reference respiratory rate, a second alarm instruction is generated, and based on the second alarm command is used to perform the second alarm operation; and otherwise, generate a first alarm instruction and a second alarm instruction at the same time, and perform a third alarm operation based on the first alarm instruction and the second alarm instruction.

In this embodiment, the partial pressure of carbon dioxide may be the subject's dynamic carbon dioxide concentration.

In this embodiment, the range of the reference partial pressure of carbon dioxide is set in advance, and is used to measure whether the current partial pressure of carbon dioxide of the subject meets the requirements.

In this embodiment, the reference breathing frequency range is set in advance, and is used to measure whether the subject's current breathing frequency meets the requirements.

In this embodiment, the first alarm instruction is used to control the alarm device to perform the first alarm operation.

In this embodiment, the first alarm operation may be one of sound alarm and light alarm.

In this embodiment, the second alarm instruction is used to control the alarm device to perform the second alarm operation.

In this embodiment, the second alarm operation may be one of sound alarm and light alarm.

In this embodiment, the third alarm operation may be a combination of sound alarm and light alarm.

The beneficial effect of the above technical solution is: by determining the partial pressure of carbon dioxide and the respiratory rate in the subject's body, and comparing them with the corresponding reference ranges, different alarm operations for different situations are realized, which is convenient for timely monitoring of the subject according to the alarm situation. Those who take corresponding measures to improve the safety of alcohol intoxication.

**Embodiment 8**

On the basis of Embodiment 1, this embodiment provides a method for solving alcohol poisoning by supplying air based on the gas mixing ratio. Set breathing device to stop working, comprising:

Extracting the alcohol concentration threshold in the safe range, and using the alcohol concentration threshold as a reference signal to generate a control instruction;
The alcohol concentration in the subject is monitored in real time, and when the alcohol concentration in the subject is equal to the alcohol concentration threshold, the control instruction is triggered to control the preset breathing device to stop working.

In this embodiment, the alcohol concentration threshold may be the maximum value of alcohol concentration in the safe range, that is, the value of the safe range is [0, alcohol concentration threshold].

In this embodiment, the reference signal is used to trigger the generation of a control instruction, and is used to control the preset breathing device to stop working.

The beneficial effect of the above technical solution is: by comparing the real-time alcohol concentration in the subject with the alcohol concentration threshold, and when the real-time alcohol concentration is equal to the alcohol concentration threshold, the preset breathing device is controlled in time to stop working, avoiding the Respiratory alkalosis improves the safety of addressing alcohol intoxication.

**Embodiment 9**

On the basis of Embodiment 1, this embodiment provides a method for solving alcohol poisoning by supplying air based on the gas mixing ratio. In Step (2), the alcohol content concentration and the current body characteristic index of the subject are input into The analysis is carried out in the alcohol analysis model, which comprises:

Read several mixed gas alcohol concentration elimination cases, and extract case data, and one mixed gas alcohol concentration elimination case corresponds to a target subject;
The case data is input into the preset neural network as sample data, and an iteration factor is determined according to the content of the sample data;
performing data iterative processing on the sample data based on the iteration factor, and determining data feature points of the sample data based on the iteration result of the preset neural network;
Wherein, the data feature points comprise: the identity information of several target subjects, the body characteristic index of the target subject and the mixing ratio of the mixed gas inhaled by the target subject;

Classify the sample data based on the data feature points, and at the same time, determine a first category vector, a second category vector, and a third category vector according to the classification result;

Respectively determine a first association relationship between the first category vector and the second category vector, a second association relationship between the first category vector and the third category vector, and the second category vector and the third category vector The third association of vectors;

Constructing the alcohol analysis model based on the first correlation, the second correlation and the third correlation;

Extracting the identity information of the subject and the body condition index of the subject, and inputting the identity information of the subject and the body condition index of the subject into the alcohol analysis model;

Based on the alcohol analysis model, determine a first weight value corresponding to the subject's identity information and a second weight value corresponding to the subject's physical condition;

In the alcohol analysis model, based on the first weight value and the second weight value, the mixing ratio of the mixed gas required by the subject is output.

In this embodiment, the case data may be the mixing ratio of carbon dioxide and oxygen or the content of carbon dioxide and oxygen used to solve the alcohol intoxication of different subjects in the case of eliminating the alcohol concentration of different mixed gases.

In this embodiment, the preset neural network is set in advance, and is used to optimize and determine optimization parameters according to sample data.

In this embodiment, the iteration factor may be reference data used for iterative processing of different sample data.

In this embodiment, the data feature points may be the identity information of several target subjects, the body characteristic index of the target subject and the mixing ratio of the mixed gas inhaled by the target subject, wherein, the target subject

The identity information of the participants is mainly the age and gender of the target subjects.

In this embodiment, the first category vector, the second category vector and the third category vector are respectively used to characterize the category of the sample data, and are determined by data feature points.

In this embodiment, the first association relationship, the second association relationship and the third association relationship are used to characterize the category association among the first category vector, the second category vector and the third category vector, so as to facilitate the construction of an alcohol analysis model.

In this embodiment, the body condition index comprises: the subject's blood pressure, heart rate, etc., which are used to characterize the subject's physical condition. The larger the index, the better the subject's physical condition.

In this embodiment, after the subject finishes eliminating the alcohol concentration based on the mixing ratio of the mixed gas, the information data corresponding to the data characteristic point of the subject is obtained, and the information data is re-input To optimize in the alcohol analysis model, the specific steps comprise:

S101: Perform normalization processing on the subject's information data, and determine the normalized information data;

$$ y(m, n, a) = [y(m, n, a)_{max} - y(m, n, a)_{min}] * \frac{x(m,n,a) - x(m,n,a)_{min}}{x(m,n,a)_{max} - x(m,n,a)_{min}}; $$

wherein $y(m, n, a)$ represents the information data after normalization; $y(m, n, a)_{max}$ represents the maximum value of sample data after normalization in the alcohol analysis model; $y(m, n, a)_{min}$ represents the minimum value of the sample data after normalization in the alcohol analysis model; $x(m, n, a)$ represents the information data without normalization; $x(m, n, a)_{min}$ represents the minimum value of sample data that is not normalized in the alcohol analysis model; $x(m, n, a)_{max}$ represents the minimum value of sample data that is normalized in the alcohol analysis model maximum value;

S102: Based on the normalized information data, iteratively optimize the fitness value of the alcohol analysis model;

$$ h = e^{1 - \ln(2.7 + \omega)} * \frac{1}{N} * \sum_{j-1}^{N} \left( \frac{Y(m,n,a)_j - y(m,n,a)}{y(m,n,a)} \right)^2 ; $$

wherein h represents the fitness value of the iterative optimization of the alcohol analysis model; $\omega$ represents the optimization factor, and the value range is (0.009, 0.010); N represents the total number of iterations; j represents the current iteration; $Y(m, n, a)_j$ represents the input of the normalized information data in the jth iteration of the alcohol analysis model out value;

S103: Comparing the fitness value with a fitness threshold, and judging whether the alcohol analysis model has completed iterative optimization;

When the fitness value is equal to or greater than the fitness threshold, it is determined that the alcohol analysis model has completed iterative optimization;

Otherwise, it is determined that the iterative optimization of the alcohol analysis model has not been completed, and step S012 is repeated until the fitness value is equal to the fitness threshold, and the iterative optimization of the alcohol analysis model is completed.

The above-mentioned fitness threshold can be set in advance, and is used to measure whether to complete the iterative optimization of the alcohol analysis model.

As mentioned above, the iterative optimization of the alcohol analysis model through the information data of the subjects is conducive to making the alcohol analysis model more accurate.

The beneficial effect of the above technical solution is: by acquiring sample data and analyzing and processing the sample data, the alcohol analysis model can be constructed accurately and quickly; secondly, the subject's identity information and body condition index are input into the alcohol analysis model for The analysis process realizes the accurate analysis of the mixing ratio of carbon dioxide and oxygen required by the subject, thus ensuring the rapid and accurate solution to the alcohol poisoning of the subject, and improving the efficiency and safety of solving alcohol poisoning.

## Embodiment 10

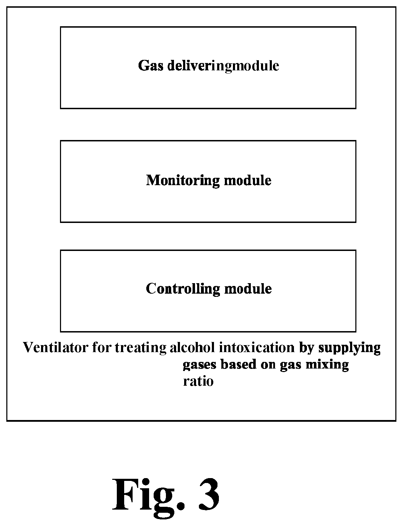

This embodiment provides a ventilator for solving alcohol poisoning based on gas mixing ratio, as shown in Fig. 3, comprising:

a gas transmission module, configured to mix oxygen and carbon dioxide to generate a target gas mixture, and transmit the target gas mixture to the subject based on the preset breathing device;

The monitoring module is used to monitor the alcohol concentration in the subject in real time, and at the same time, monitor the partial pressure of carbon dioxide and the respiratory rate in the subject;

A control module, configured to control the preset breathing device to stop working when the alcohol concentration in the subject meets a safe range on the basis of the subject's partial pressure of carbon dioxide and respiratory rate being stable.

The beneficial effect of the above-mentioned technical solution is: the mixed gas supply of carbon dioxide $CO_2$ and oxygen $O_2$ in different proportions can quickly remove the alcohol in the body, and at the same time avoid respiratory alkalosis caused by hyperventilation, and improve the efficiency and safety of solving alcohol intoxication.

Obviously, those skilled in the art can make various changes and modifications to the present invention without departing from scope of the present invention as defined by the following appended claim.

## Claims

1. A ventilator for treating alcohol intoxication based on gas mixing ratio, comprising:

a gas transmission module configured to mix oxygen and carbon dioxide to generate a target gas mixture, and transmit the target gas mixture to a subject based on a preset breathing device;

a monitoring module configured to monitor an alcohol concentration in the subject in real time, and meanwhile, monitor a partial pressure of carbon dioxide and a respiratory rate in the subject;

a control module configured to control the preset breathing device to stop working when the alcohol concentration in the subject meets a safe range and on the basis of the partial pressure of the carbon dioxide and the respiratory rate of the subject being stable;

wherein in the gas transmission module, mixing the oxygen and the carbon dioxide to generate the target mixed gas, comprising:

collecting a current alcohol concentration of the subject, and at the same time, determining current body characteristic indexes of the subject, wherein the current body characteristic index comprises: the subject's blood pressure and heart rate, which are used to characterize the subject's physical condition;

inputting the current alcohol content concentration of the subject and the current body characteristic indexes of the subject into an alcohol analysis model for analysis;

based on analysis results in the alcohol analysis model, determining an oxygen concentration and a carbon dioxide concentration that the subject needs respectively; based on the oxygen concentration and the carbon dioxide concentration that the subject needed respectively, determining a mixing ratio of the oxygen and carbon dioxide;

based on the mixing ratio, mixing the oxygen and the carbon dioxide to generate the target mixed gas;

wherein in the monitoring module, monitoring the alcohol concentration in the subject's body in real time, and at the same time, monitoring the partial pressure of carbon dioxide and the respiratory rate of the subject, comprising:

> when the target mixed gas is transmitted to the subject based on the preset breathing device, start a first monitoring instruction and a second monitoring instruction;
> monitoring a dynamic alcohol concentration in the subject in real time based on the first monitoring instruction;
> monitoring a partial pressure of carbon dioxide in the subject and the respiratory rate of the subject in real time based on the second monitoring instruction;
> according to a preset time point, dynamic alcohol change concentrations are recorded in one-to-one correspondence with the partial pressure of the carbon dioxide and the respiratory rate in the subject;
> based on recorded results, generating a monitoring dynamic table and displaying in real time.

**Patentansprüche**

1. Beatmungsgerät zur Behandlung einer Alkoholintoxikation auf der Grundlage eines Gasmischungsverhältnisses, umfassend:

> ein Gasübertragungsmodul, das so eingerichtet ist, dass es Sauerstoff und Kohlendioxid mischt, um ein Zielgasgemisch zu erzeugen, und das Zielgasgemisch an einen Probanden auf der Grundlage eines voreingestellten Beatmungsgeräts überträgt;
> ein Überwachungsmodul, das so eingerichtet ist, dass es eine Alkoholkonzentration im Probanden in Echtzeit überwacht und gleichzeitig einen Kohlendioxidpartialdruck und eine Atemfrequenz im Probanden überwacht;
> ein Steuermodul, das so eingerichtet ist, dass es die voreingestellte Atemvorrichtung steuert, um ihre Funktion zu beenden, wenn die Alkoholkonzentration im Probanden einen sicheren Bereich erreicht und basierend auf dem Partialdruck des Kohlendioxids und der Atemfrequenz des Probanden stabil ist;
> wobei im Gasübertragungsmodul das Mischen des Sauerstoffs und des Kohlendioxids zur Erzeugung des Zielgemischgases erfolgt, umfassend:

>> Erfassen einer aktuellen Alkoholkonzentration des Probanden und gleichzeitiges Bestimmen aktueller Körperkennzahlen des Probanden, wobei die aktuelle Körperkennzahl umfasst: den Blutdruck und die Herzfrequenz des Probanden, die zur Charakterisierung des körperlichen Zustands des Probanden verwendet werden;
>> Eingabe der aktuellen Alkoholkonzentration des Probanden und der aktuellen Körperkennzahlen des Probanden in ein Alkoholanalysemodell zur Analyse;
>> basierend auf Analyseergebnissen im Alkoholanalysemodell, Bestimmung einer Sauerstoffkonzentration und einer Kohlendioxidkonzentration, die der Proband jeweils benötigt; basierend auf der Sauerstoffkonzentration und der Kohlendioxidkonzentration, die der Proband jeweils benötigt, Bestimmung eines Mischungsverhältnisses von Sauerstoff und Kohlendioxid;
>> basierend auf dem Mischungsverhältnis, das Mischen von Sauerstoff und Kohlendioxid zur Erzeugung des Zielgemischgases;
>> wobei im Überwachungsmodul das Überwachen der Alkoholkonzentration im Körper des Probanden in Echtzeit und gleichzeitig das Überwachen des Partialdrucks von Kohlendioxid und der Atemfrequenz des Probanden erfolgt, umfassend:

>>> wenn das Zielgasgemisch dem Patienten basierend auf dem voreingestellten Beatmungsgerät zugeführt wird, das Starten einer ersten Überwachungsanweisung und einer zweiten Überwachungsanweisung;
>>> Überwachung einer dynamischen Alkoholkonzentration des Probanden in Echtzeit auf der Grundlage der ersten Überwachungsanweisung;
>>> Überwachung eines Kohlendioxidpartialdrucks im Probanden und der Atemfrequenz des Probanden in Echtzeit auf der Grundlage der zweiten Überwachungsanweisung;

nach einem voreingestellten Zeitpunkt werden die dynamischen Alkoholveränderungskonzentrationen in 1:1-Korrespondenz mit dem Partialdruck des Kohlendioxids und der Atemfrequenz des Probanden aufgezeichnet;

basierend auf den aufgezeichneten Ergebnissen wird eine dynamische Überwachungstabelle erstellt und in Echtzeit angezeigt.

**Revendications**

1. Ventilateur pour le traitement d'une intoxication alcoolique sur la base d'un rapport de mélange de gaz, comprenant :

un module de transmission de gaz configuré pour mélanger de l'oxygène et du dioxyde de carbone pour générer un mélange de gaz cible, et transmettre le mélange de gaz cible à un sujet sur la base d'un dispositif respiratoire préréglé ;

un module de surveillance configuré pour surveiller une alcoolémie chez le sujet en temps réel, et entre-temps, surveiller une pression partielle de dioxyde de carbone et une fréquence respiratoire chez le sujet ;

un module de commande configuré pour commander le dispositif respiratoire préréglé pour qu'il cesse de fonctionner lorsque l'alcoolémie chez le sujet atteint une plage de sécurité et sur la base du fait que la pression partielle du dioxyde de carbone et la fréquence respiratoire du sujet sont stables ;

dans lequel, dans le module de transmission de gaz, le mélange de l'oxygène et du dioxyde de carbone pour générer le gaz mélangé cible, comprenant :

la collecte d'une alcoolémie actuelle du sujet, et dans le même temps, la détermination d'indices caractéristiques corporels actuels du sujet, dans lequel l'indice caractéristique corporel actuel comprend : la tension artérielle et la fréquence cardiaque du sujet, qui sont utilisées pour caractériser la condition physique du sujet ;

l'entrée de l'alcoolémie actuelle du sujet et des indices caractéristiques corporels actuels du sujet dans un modèle d'analyse d'alcool pour analyse ;

sur la base des résultats d'analyse dans le modèle d'analyse d'alcool, la détermination d'une concentration en oxygène et d'une concentration en dioxyde de carbone dont le sujet a besoin respectivement ; sur la base de la concentration en oxygène et de la concentration en dioxyde de carbone dont le sujet a besoin respectivement, la détermination d'un rapport de mélange de l'oxygène et du dioxyde de carbone ;

sur la base du rapport de mélange, le mélange de l'oxygène et du dioxyde de carbone pour générer le gaz mélangé cible ; dans lequel, dans le module de surveillance, la surveillance de l'alcoolémie dans l'organisme du sujet en temps réel, et dans le même temps, la surveillance de la pression partielle de dioxyde de carbone et de la fréquence respiratoire du sujet, comprenant :

lorsque le gaz mélangé cible est transmis au sujet sur la base du dispositif respiratoire préréglé, le démarrage d'une première instruction de surveillance et d'une deuxième instruction de surveillance ;

la surveillance d'une alcoolémie dynamique chez le sujet en temps réel sur la base de la première instruction de surveillance ;

la surveillance d'une pression partielle de dioxyde de carbone chez le sujet et de la fréquence respiratoire du sujet en temps réel sur la base de la deuxième instruction de surveillance ;

selon un instant prédéfini, des changements d'alcoolémie dynamique sont enregistrés en correspondance biunivoque avec la pression partielle du dioxyde de carbone et la fréquence respiratoire du sujet ;

sur la base des résultats enregistrés, la génération d'un tableau dynamique de surveillance et l'affichage en temps réel.

Step (1)

Mixing the oxygen and the carbon dioxide to generate a target mixed gas mixture, and transmitting the target gas mixture to a subject based on a preset breathing device;

Step (2)

Monitoring the alcohol concentration in the subject's body in real time, and at the same time, monitoring the partial pressure of carbon dioxide and respiratory rate in the subject's body

Step (3)

On the basis that the partial pressure of carbon dioxide and respiratory rate of the subject are stable,

when the alcohol concentration in the subject meets the safety range, controlling the preset ventilator to stop working

# Fig. 1

**Fig. 2**

Gas deliveringmodule

Monitoring module

Controlling module

Ventilator for treating alcohol intoxication by supplying
gases based on gas mixing
ratio

# Fig. 3

# Fig. 4

TC=Treatment

groupCG=Control group

# Fig. 5

TG=Treatment group

CG=Control group

# Fig. 6

TG=Treatment group
CG=Control group

# Fig. 7

**Fig. 8**

TG=Treatment group

CG=Control group

TG=Treatment group

CG=Control group

# Fig. 9

Awake time

TG=Treatment group

CG=Control group

# Fig. 10

# EP 4 292 634 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 106139343 A
- CN 111658932 A
- US 3974830 A
- CN 208710724 U
- CN 105748069 A
- SU 1296157 A1